# EUROPEAN PATENT SPECIFICATION

(11) **EP 1 850 893 B1**
(45) Date of publication and mention of the grant of the patent: **26.11.2008**
(21) Application number: 06735839.0
(22) Date of filing: 22.02.2006
(51) Int. Cl.: A61M 5/315, A61M 5/145

(54) **BACKER PLATE ADAPTER FOR A SYRINGE PLUNGER**
STÜTZPLATTENADAPTER FÜR EINEN SPRITZENKOLBEN
ADAPTATEUR DE PLAQUE D'APPUI POUR PISTON DE SERINGUE

(30) Priority: 23.02.2005 US 655359 P
(43) Date of publication of application: 07.11.2007
(73) Proprietor: MALLINCKRODT, INC., St. Louis Missouri 63134 (US)
(72) Inventor: BRUCE, John, Kevin, Burlington, Kentucky 41005 (US)
(74) Representative: Chettle, Adrian John
(86) International application number: PCT/US2006/006336
(87) International publication number: WO 2006/091695

(56) References cited:
- US-A- 4 636 198
- US-A- 5 865 805
- US-A1- 2004 116 893
- US-B1- 6 312 410
- US-B1- 6 336 913

## Description

### FIELD OF INVENTION

The present invention relates generally to injectors for injecting fluid into animals, and particularly to a backer plate adapter for a syringe plunger in an injector.

### BACKGROUND

During many medical procedures, various fluids are injected into patients for purposes of diagnosis or treatment. An example of one such fluid is contrast media used to enhance angiography or CT imaging. The injectors used in these procedures are automated devices that expel the fluid from a syringe, through a tube, and into the subject.

Injectors suitable for these applications must have relatively large volume syringes and be capable of producing relatively large flow rates and injection pressures. For this reason, injectors for such applications typically include large, high mass injector motors and drive trains. These are typically housed in an injection head, which is supported by a floor-, wall-, or ceiling-mounted arm.

The syringes used in these injectors generally include a barrel with a hollow interior and a discharge tip, and a syringe plunger disposed within the barrel. The rearward face of the syringe plunger of many of these syringes is formed by a backer plate. The backer plate generally includes a first portion that supports a rubber cap to form the syringe plunger, and a second portion including a coupling element, which is engageable with the plunger drive ram. In many backer plates, the coupling element is an extension referred to as a "button," and consists of a shaft portion protruding from the rearward face of the backer plate, topped by a cap portion. When the new syringe, including syringe plunger and associated backer plate, is inserted, the button contacts a plunger-coupling element, such as engagement jaws, located on the forward end of the plunger drive ram. These jaws then snap around the button of the backer plate, thereby connecting the backer plate and syringe plunger to the plunger drive ram.

In some cases, it is necessary to add an adapter to the backer plate to, e.g., reduce the volume of a prefilled syringe. In one embodiment, shown in U.S. Patent No. 4,636,198, this is accomplished by a backer plate adapter having jaws at its first end for gripping a syringe plunger button, and a similar button at its second end to be gripped by a plunger drive ram. Other embodiments of such an adapter are also known. For example, the rearward face of the syringe plunger is often threaded to mate with complementary threads located on a first end of a backer plate adapter. The backer plate adapter is then installed to the syringe plunger by mating the threads and rotating the backer plate adapter relative to the threaded syringe plunger. This is oftentimes done with the syringe plunger already located within the barrel of the syringe. Thus, much of the backer plate adapter being installed may also be disposed within the syringe barrel during installation. As a result, in order to properly connect the backer plate adapter to the syringe plunger, a user must grip the button in order to rotate the backer plate adapter. However, the button is small and can be difficult to manipulate by hand. Historically, the cap portion of the button on the backer plate adapter has been a circular shape. This shape and the size of the button make it very difficult to tighten or loosen the backer plate adapter from the syringe plunger.

Additionally, due to the difficulty of reaching inside a syringe barrel to rotate the backer plate adapter, and thus tighten or loosen the backer plate adapter from the syringe plunger, tools have been developed to help grip the button to rotate the backer plate adapter. However, as described above, historically the buttons have been clrcular In shape, and thus are difficult to grip, even with a tool. Further, the tools remain separate from the syringe and InJector systems, and thus are easy to misplace.

In view of the above, it would be desirable to provide a backer plate adapter that may be easily mechanically tightened or loosened from a syringe plunger, even when the backer plate adapter Is positioned within the barrel of a syringe. Further, It would be desirable to provide a backer plate adapter that may be tightened or loosened without the use of a separate tool. With this background In mind, we now turn to a summary of the present Invention.

US-A-6336913 discloses a syringe backer plate adapter having the features of the pre-characterizing clause of claim 1 appended hereto.

### SUMMARY

The present invention overcomes the drawbacks described above, in one aspect, by providing a backer plate adapter that Includes a button extension having a cap portion exhibiting a noncircular shape, such as a hexagon shape, that facilitates mechanically tightening or loosening the backer plate, adapter from a syringe plunger. Further, the backer plate adapter may Include a recess having the same, or substantially the same, noncircular shape, such as a hexagon shape, In the end of the backer plate adapter opposite the button. Thus, when provided a plurality of backer plate adapters, one backer plate adapter may be used as a tool to assemble or disassemble another backer plate adapter from a syringe plunger by using the recess of one backer plate adapter to engage the cap portion of the other backer plate adapter. The dimensions of the body portion of the adapter, for example the diameter, may be larger than the button. This aids in using the adapter as a tool when gripping the body. Backer plate adapters may be disposable, and therefore are generally ordered In quantities greater than a single backer plate adapter. So, In the above scenario, as long as a user has at least one extra backer plate adapter, the user has a tool to Install and remove another backer plate_ adapter from the syringe plunger.

In particular, the present invention includes a backer plate adapter for a syringe plunger that includes a first end and a second end. The first end of the backer plate adapter is adapted to be engaged with a syringe plunger. A portion of that first end defines a recess. The second end of the backer plate adapter includes an extension protruding therefrom. This extension includes a shaft portion and a cap portion. A cross-section of the cap portion, taken transverse to the longitudinal axis of the extension, has a first shape, such as a hexagon. The recess has a shape that is complementary to the first shape. The shape of the recess therefore may be the same as, or substantially the same as, the first shape of the cap portion. Thus, the recess may also have the first shape.

Since, as described above, the backer plate adapters are generally provided as pluralities of backer plate adapters, the present invention, In another aspect, may also comprise a second backer plate adapter having first and second ends Including a recess and extension, respectively. The recess of the second backer plate adapter has a shape that is complementary to the first shape of the cap portion of the first backer plate adapter, as described above. Thus, the recess of the second backer plate adapter may have the first shape. Likewise, the extension of the second backer plate adapter includes a shaft portion and a cap portion, and a transverse cross-section of the cap portion of the second backer plate adapter may also have a complementary shape, such as the first shape.

In another aspect, the present invention further includes an injection system, having a backer plate adapter, or plurality of backer plate adapters, as described above. Such an injection system more specifically includes a syringe including a barrel, a syringe plunger adapted to be disposed within the barrel, and a first backer plate adapter having a first end adapted to be engaged with the syringe plunger, and a second end including an extension protruding therefrom. At least a portion of the first end of the backer plate adapter defines a recess. The extension includes a shaft portion and a cap portion, wherein a cross-section of the cap portion has a first shape. The injection system may also include a second backer plate adapter including a recess at one end, wherein that recess has a shape complementary to the first shape.

By use of these pluralities of backer plate adapters, the present invention also includes a method of operatively connecting a backer plate adapter to, or removing a backer plate adapter from, a syringe plunger. This method of operatively connecting a backer plate adapter to a syringe plunger includes the following steps. First, a first backer plate adapter, as described above, is placed in proximity to a syringe plunger such that threads on the first backer plate adapter are aligned to mate with threads on the syringe plunger. Next, one would take a second backer plate adapter, as described above, and use the recess on the first end of the second backer plate adapter as a tool to engage the cap portion, such as a hexagon-shaped cap portion, on the extension of the second end of the first backer plate adapter. Once the recess has engaged the cap of the extension, the second backer plate adapter may be rotated relative to the syringe plunger, thereby cooperatively rotating the first backer plate adapter such that its threads will mate with the threads of the syringe plunger. The method of removing the first backer plate adapter from the syringe plunger includes rotating the second backer plate adapter in an opposite direction to cooperatively rotate the first backer plate adapter in the opposite direction, such that the first backer plate adapter may be disengaged from the syringe plunger.

Advantages of the present invention will be apparent to those skilled in the art in conjunction with the Detailed Description below, and the drawings attached to this application.

### BRIEF DESCRIPTION OF THE DRAWINGS

Fig. 1A is a perspective view of one embodiment of a backer plate adapter in accordance with the principles of the present invention, depicting an end of the backer plate adapter including an extension;
Fig. 1B is a perspective view of the backer plate adapter of Fig. 1A in accordance with the principles of the present invention, depicting an end of the backer plate adapter including a recess;
Fig. 1C is a diagrammatic cross-section of the embodiment of Fig. 1A taken along line 1C-1C of Fig. 1A;
Fig. 1D is a diagrammatic cross-section of the embodiment of Fig. 1A taken along line 1D-1D of Fig. 1B;
Fig. 2A is an end view of the cap portion of an extension of a backer plate adapter in accordance with the principles of the present invention;
Fig. 2B is an end view of a recess in one embodiment of the backer plate adapter of Fig. 2A in accordance with the principles of the present invention;
Fig. 3 is a partial cross-sectional view of first and second backer plate adapters engaged with one another in accordance with the principles of the present invention;
Fig. 3A is a diagrammatic cross-section of the embodiment of Fig. 1A taken along line 3A-3A of Fig. 3;
Fig. 3B is a diagrammatic cross-section of the embodiment of Fig. 1A taken along line 3B-3B of Fig. 3;
Fig. 4 is a partial cross-sectional view of a syringe and syringe plunger to be used with a backer plate adapter of the present invention, depicting the threaded rearward end of the syringe plunger;
Fig. 5 is a partial cross-sectional view of a syringe, syringe plunger, a first backer plate adapter in accordance with the principles of the present invention positioned to engage the syringe plunger, and a second backer plate adapter, which may be used to engage the first backer plate adapter;
Fig. 6 is a partial cross-sectional view of a syringe, syringe plunger engaged with a first backer plate adapter in accordance with the principles of the present invention, and a second backer plate adapter engaged with the first backer plate adapter; and
Fig. 7 is a perspective view of one embodiment of a backer plate adapter in accordance with the principles of the present invention, depicting the backer plate adapter having a push rod to be used for manual injections.

### DETAILED DESCRIPTION

Referring to the Figures, the present invention, in one aspect, provides a backer plate adapter 10 that includes a button, also referred to as an extension 12, including a cap portion 16 having a noncircular shape that facilitates mechanically tightening or loosening the backer plate adapter 10 from a syringe plunger 20. Further, the backer plate adapter 10 may include a recess 22 having the same or substantially the same noncircular shape, such as a hexagon shape, in the end of the backer plate adapter 10 opposite the extension 12. Thus, when a plurality of backer plate adapters are provided, a second backer plate adapter 10' may be used as a tool to assemble or disassemble a first backer plate adapter 10 from a syringe plunger 20 by using the recess 22' of the second backer plate adapter 10' to engage the cap portion 16 of the first backer plate adapter 10. Backer plate adapters may be disposable, and therefore are generally ordered in quantities greater than a single backer plate adapter. So, in the above scenario, as long as a user has at least one extra backer plate adapter, the user has a tool to install and remove another backer plate adapter from the syringe plunger. While the backer plate adapters may be disposable, it will be recognized that alternatively the backer plate adapters may be reused, since they do not come into contact with fluid in the syringe, and thus do not need to be sterile.

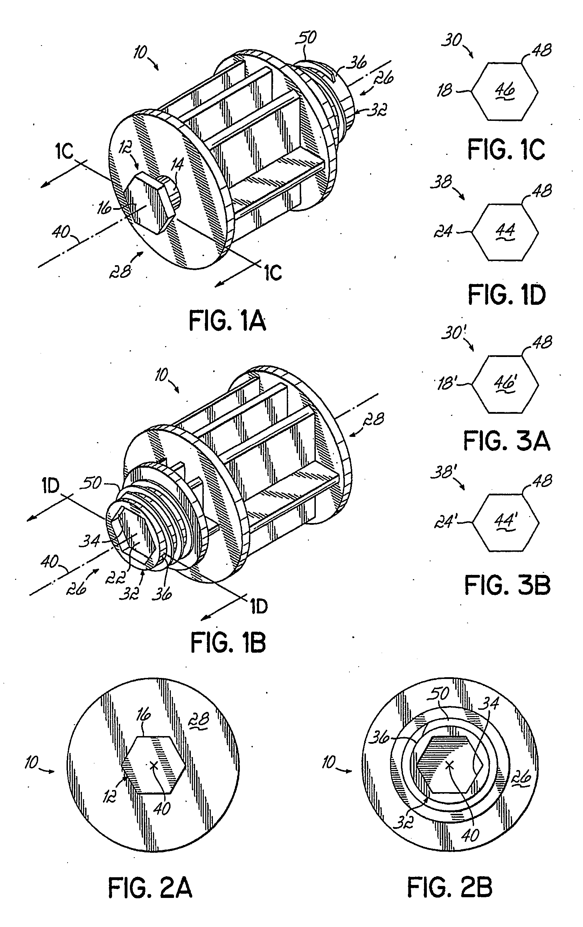

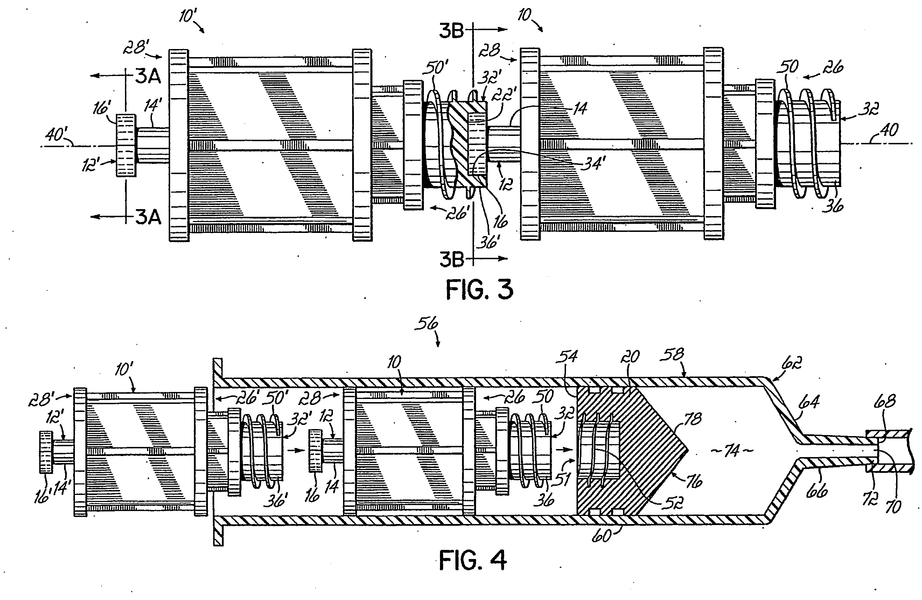

In particular, and referring to Figs. 1A-2B, the present invention includes a backer plate adapter 10 that includes a first end 26 and a second end 28. The first end 26 of the backer plate adapter 10 is adapted to be engaged with a syringe plunger 20 (see Fig. 4). A portion of that first end 26 defines a recess 22. The second end 28 of the backer plate adapter 10 includes an extension 12 protruding therefrom. This extension 12 includes a shaft portion 14 and a cap portion 16. Referring to Fig. 1C, a cross-section 30 of the cap portion 16 of this second end 28, taken transverse to a longitudinal axis 40 of the backer plate adapter 10, has a first shape 18. The recess 22 has a shape 24 that is complementary to the first shape 18. The shape 24 of the recess 22 therefore may be the same as, or substantially the same as, the first shape 18 of the cross-section 30 of the cap portion 16. Thus, the recess 22 may also have the first shape 18. The relative sizes of the recess 22 and the cap portion 16 may differ.

Referring to Figs. 1A and 2A, the second end 28 of the backer plate adapter 10 includes an extension 12. The extension 12 on the second end 28 of the backer plate adapter 10 includes a shaft portion 14 and a cap portion 16. The cap portion 16 exhibits a first shape 18. More specifically, a cross-section 30 of the cap portion 16 taken transverse to the longitudinal axis 40 of the backer plate adapter 10 exhibits this first shape 18. This first shape 18 may be quadrilateral, square, rectangular, trapezoidal, pentagonal, hexagonal, or octagonal, for example. It will be recognized by those of skill in the art that the first shape of the cap portion 16 may be of shapes other than those specifically recited above.

Referring now to Figs. 1B and 2B, the first end 26 of the backer plate adapter 10 includes at least one wall 32 defining the recess 22. More specifically, in the illustrated embodiment, the wall 32 extends at the first end 26 of the backer plate adapter 10 and includes an inner wall surface 34 and an outer wall surface 36. The inner wall surface 34 defines a portion of the outer boundaries of the recess 22. Referring to Fig. 1D, a cross-section 38 of the portion of the first end 26, including the wall 32 and recess 22, taken transverse to the longitudinal axis 40 of the backer plate adapter 10, exhibits a shape 24 complementary to the first shape 18 of the cap portion 16. The complementary shape 24 of the recess 22 may be the same as, or substantially the same as, the first shape 18 of the cap portion 16. Thus, the recess 22 may also have the first shape 18. The complementary shape 24 of the recess 22 may be quadrilateral, square, rectangular, trapezoidal, pentagonal, hexagonal, or octagonal. It will be recognized by those of skill in the art that the complementary shape 24 of the recess 22 may be of shapes other than those specifically recited above.

With reference to Figs. 1A-2B, the recess 22 on the first end 26 of the backer plate adapter 10 and the cap portion 16 of the extension 12 on the second end 28 of the backer plate adapter 10 each exhibit the same first shape 18 in the illustrated embodiment. While the shapes 24, 18 of the recess 22 and cap portion 16 are complementary, and may be the same or substantially the same, it is not necessary that the recess 22 and cap portion 16 be of the same size (i.e., exhibit the same area in their cross-sections), although they may. In the illustrated embodiment, the first shape 18 of the recess 22 is a first size, and the complementary shape 24 of the cap portion 16 is a second size.

More specifically, the cross-section 38 of the portion of the first end 26 taken transverse to the longitudinal axis 40 of the backer plate adapter 10 exhibits a first area 44, and the cross-section 30 of the cap portion 16 taken transverse to the longitudinal axis 40 of the backer plate adapter 10 exhibits a second area 46. In one embodiment, the first area 44 is a different size than said second area 46. In particular, the first area 44 may be larger than the second area 46. In an alternate embodiment, the first area 44 is substantially equal to the second area 46.

Since, as described above, the backer plate adapters are generally provided as pluralities of backer plate adapters, the present invention, in another aspect, may also comprise a second backer plate adapter 10' having first and second ends 26', 28' including a recess 22' and extension 12', respectively (see Fig. 3). In the illustrated embodiment, the recess 22' of the second backer plate adapter 10' has a shape 24' that is complementary to the first shape 18 of the cap portion 16 of the first backer plate adapter 10, described above. Thus, the recess 22' of the second backer plate adapter 10' may have the first shape 18. Likewise, the extension 12' of the second backer plate adapter 10' includes a shaft portion 14' and a cap portion 16', and a transverse cross-section 30' (Fig. 3A) of the cap portion 16' of the second backer plate adapter 10' may also have a complementary shape 18', such as the first shape 18.

Referring to Figs. 1A-3, the plurality of backer plate adapters of the illustrated embodiment of the invention more specifically includes a first backer plate adapter 10 and a second backer plate adapter 10'. The first backer plate adapter 10 includes a first end 26 and a second end 28, wherein the second end 28 includes an extension 12 protruding therefrom. This extension 12 includes a shaft portion 14 and a cap portion 16. The second backer plate adapter 10' includes a first end 26' and a second end 28'. A portion of the first end 26' of the second backer plate adapter 10' defines a recess 22'. The recess 22' defined by the first end 26' has a shape 24' that is complementary to the first shape 18 of the cap portion 16 of the first backer plate adapter 10. The shape 24' of the recess 22' may be the same as, or substantially the same as, the first shape 18 of the cap portion 16. Thus, in one embodiment, the recess 22' and cap portion 16 may each have the first shape 18. This first shape 18 may be quadrilateral, square, rectangular, trapezoidal, pentagonal, hexagonal, or octagonal, for example. It will be recognized by those of skill in the art that the first shape 18 may be of shapes other than those specifically recited above.

As described above, the recess 22' of the second backer plate adapter 10' is of a complementary shape 24' to the first shape 18. In order to form this complementary shape 24' in the recess 22' of the second backer plate adapter 10', the first end 26' of the second backer plate adapter 10' includes at least one wall 32' defining the recess 22'. More specifically, in the illustrated embodiment, the wall 32' extends at the first end 26' of the second backer plate adapter 10' and includes an inner wall surface 34' and an outer wall surface 36'. The inner wall surface 34' defines a portion of the outer boundaries of the recess 22'. A cross-section 38' (Fig. 3B) of the portion of the first end 26' including the wall 32' and recess 22' of the second backer plate adapter 10', taken transverse to the longitudinal axis 40' of the second backer plate adapter 10', exhibits the complementary shape 24'. In particular, in use, the first and second backer plate adapters 10, 10' are adapted to engage one another with the cap portion 16 of the first backer plate adapter 10 being received by the recess 22' of the second backer plate adapter 10'. Thus, in the engagement of the first and second backer plate adapters 10, 10' one to another, it will be understood by those of skill in the art that any complementary shapes that allow for cooperative rotation of the first backer plate adapter 10 upon rotation of the second backer plate adapter 10' will suffice. Thus, in certain embodiments, the complementary shapes may each be noncircular. The complementary shapes of the recess and cap portion therefore each include at least one noncircular portion, such as at least one corner edge 48, to allow for cooperative rotation of the first and second backer plate adapters 10, 10'.

While the first shape 18 of the cap portion 16 and the shape 24' of the recess 22' are complementary, and may be the same or substantially the same, it is not necessary that the recess 22' and cap portion 16 be of the same size, although they may be. In the illustrated embodiment, the shape 24' of the recess 22' of the second backer plate adapter 10' is a first size, and the first shape 18 of the cap portion 16 of the first backer plate adapter 10 is a second size. The recess 22' of the second backer plate adapter 10' and the cap portion 16 of the first backer plate adapter 10 are adapted to engage one another. As described above, the relative sizes of the recess and cap portion may be different. However, since the second backer plate adapter 10' is used as a tool to rotate the first backer plate adapter 10, it will be understood by those skilled in the art that the sizes may only be different such that the recess 22' and cap portion 16 are still functional to engage one another such that the first and second backer plate adapters 10, 10' may cooperatively rotate when engaged.

More specifically regarding the relative sizes of the recess 22' and cap portion 16, the cross-section 38' of the portion of the first end 26' taken transverse to the longitudinal axis of the first end 26' exhibits a first area 44' of the recess 22' of the second backer plate adapter 10'. The cross-section 30 of the cap portion 16 of the first backer plate adapter 10, taken transverse to the longitudinal axis of the extension 12, exhibits a second area 46. In one embodiment, the first area 44' is a different size than said second area 46. In particular, the first area 44' may be larger than said second area 46. In an alternate embodiment, the first area 44' is substantially equal to the second area 46. The relative sizes of the first area 44' and second area 46, like the complementary shapes 18, 24' described above, are such as to allow for engagement and/or cooperative rotation of the first and second backer plate adapters 10, 10'.

Like the first end 26' of the second backer plate adapter 10', the first end 26 of the first backer plate adapter 10 may define a recess 22 having a shape 24 complementary to the first shape 18 of the cap portion 16 of the first backer plate adapter 10. In the illustrated embodiment, the recess 22 of the first backer plate adapter 10 has the first shape 18. Thus, the first end 26 may include at least one wall 32 defining the recess 22. More specifically, the wall 32 extends at the first end 26 of the backer plate adapter and includes an inner wall surface 34 and an outer wall surface 36. The inner wall surface 34 defines a portion of the outer boundaries of the recess 22. A cross-section 38 of the portion of the first end 26 including the wall 32 and recess 22 of the first backer plate adapter 10, taken transverse to the longitudinal axis 40 of the first backer plate adapter 10, exhibits the first shape 18.

The first end 26 of the first backer plate adapter 10 is adapted to engage a syringe plunger 20. In the illustrated embodiment, the first end 26 of the first backer plate adapter 10 includes threads 50. These threads 50 are complementary to threads 52 that are disposed on the rearward face 54 of the syringe plunger 20. The first backer plate adapter 10 and syringe plunger 20 may engage one another by mating the threads 50 of the first backer plate adapter 10 with the complementary threads 52 of the syringe plunger 20. Like that of the first backer plate adapter 10, the first end 26' of the second backer plate adapter 10' may also be adapted to engage a plunger. In the illustrated embodiment, the first end 26' of the second backer plate adapter 10' includes threads 50'. These threads 50' are adapted to mate with complementary threads 52 disposed on a syringe plunger 20. In the illustrated embodiment, the first end 26 of the first backer plate adapter 10 more specifically includes outer threads 50 disposed on its outer surface, and the syringe plunger 20 includes a cavity 51 having internal threads 52. This cavity 51 and internal threads 52 receive the first end 26 and external threads 50. However, it will be recognized by those of skill in the art that this configuration is merely exemplary, and other configurations are consistent with the principles of the present invention. For example, the first end of the backer plate adapter may include a cavity with internal threads and the rearward face of the syringe plunger may include a protrusion with external threads.

Like the first backer plate adapter 10, the second backer plate adapter 10' may include an extension 12' on its second end 28'. The extension 12' on the second end 28' of the second backer plate adapter 10' includes a shaft portion 14' and a cap portion 16'. The cap portion 16' may also have a shape 18' that is complementary to the first shape 18 of the cap portion 16 of the first backer plate adapter 10. More specifically, a cross-section 30' of the cap portion 16' taken transverse to the longitudinal axis 40' of the second backer plate adapter 10' exhibits this complementary shape 18'.

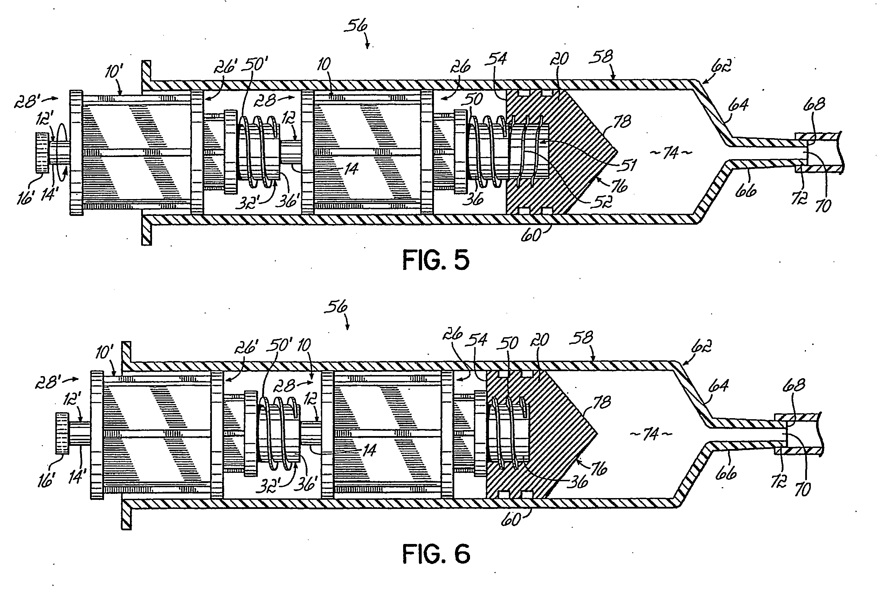

Referring now to Figs. 4-6, the present invention further includes an injection system 56, having a backer plate adapter, or plurality of backer plate adapters, as described above. Such an injection system 56 more specifically includes a syringe 58 including a barrel 60, a syringe plunger 20 adapted to be disposed within the barrel 60, and a first backer plate adapter 10 having a first end 26 adapted to be engaged with the syringe plunger 20, and a second end 28 including an extension 12 , protruding therefrom. The extension 12 includes a shaft portion 14 and a cap portion 16, wherein a cross-section 30 of the cap portion 16 has a first shape 18. At least a portion of the first end 26 of the backer plate adapter defines a recess 22. The recess 22 defined by the first end 26 may have a shape 24 complementary to the first shape 18. The shape 24 of the recess 22 may be the first shape 18.

As is well known to those skilled in the art, and with reference to Fig. 4, the syringe 58 includes an exterior cylindrical barrel 60 or body, which at its forward end 62 is integral with a conical front wall section 64. A neck 66, terminating in discharge tip 68, extends forwardly from and is integral with the front wall. The neck 66 of the discharge tip 68 contains an orifice 70 in its remote end 72 that communicates with an internal syringe cavity 74 formed within the neck 66, the conical front wall and the cylindrical barrel 60 of the syringe 58. The rear end 76 of the cavity 74 is further defined by a forward facing conical surface 78 of the syringe plunger 20. The conical surface 78 is of a slope that conforms to the slope of the interior of the conical front wall. The syringe plunger 20 is snugly slidable within the body of the syringe case such that the cavity 74 is of variable volume. The rearward facing side of the syringe plunger 20 includes threads 52, in the illustrated embodiment. These threads 52 mate with threads 50 disposed on a backing plate, such that the backing plate and syringe plunger 20 may engage one another.

Still referring to Figs. 4-6, the injection system 56 may further include a second backer plate adapter 10'. As described above with respect to the plurality of backer plate adapters, the second backer plate adapter 10' includes first and second ends 26', 28' including a recess 22' and extension 12', respectively. In the illustrated embodiment, the recess 22' has a shape 24' that is complementary to the first shape 18 of the cap portion 16 of the first backer plate adapter 10. Thus, the recess 22' of the second backer plate adapter 10' may have the first shape 18. Likewise, the extension 12' of the second backer plate adapter 10' includes a shaft portion 14' and a cap portion 16', and a transverse cross-section 30' of the cap portion 16' of the second backer plate adapter 10' may also have a complementary shape 18', such as the first shape 18.

In order to form the complementary shape 24' in the recess 22' of the second backer plate adapter 10', the first end 26' of the second backer plate adapter 10' includes at least one wall 32' defining the recess 22'. More specifically, in the illustrated embodiment, the wall 32' extends at the first end 26' of the second backer plate adapter 10' and includes an inner wall surface 34' and an outer wall surface 36'. The inner wall surface 34' defines a portion of the outer boundaries of the recess 22'. A cross-section 38' of the portion of the first end 26' including the wall 32' and recess 22' of the second backer plate adapter 10', taken transverse to the longitudinal axis 40' of the second backer plate adapter 10', exhibits the complementary shape 24'. The shape 24' may be quadrilateral, square, rectangular, trapezoidal, pentagonal, hexagonal, or octagonal, for example. It will be recognized by those of skill in the art that the shape 24' of the recess 22' may be of shapes other than those specifically recited above.

In use, the first and second backer plate adapters 10, 10' are adapted to engage one another with the cap portion 16 of the first backer plate adapter 10 being received by the recess 22' of the second backer plate adapter 10'. Thus, in the engagement of the first and second backer plate adapters 10, 10' one to another, it will be understood by those of skill in the art that any complementary shapes 24', 18 that allow for cooperative rotation of the first backer plate adapter 10 upon rotation of the second backer plate adapter 10' will suffice. Thus, in certain embodiments, the complementary shapes may include at least one noncircular portion, such as at least one corner edge 48, to allow for cooperative rotation of the first and second backer plate adapters 10, 10'.

With reference to Figs. 1-6, while the first shape 18 of the cap portion 16 and shape 24' of the recess 22' are complementary, and may be the same or substantially the same, it is not necessary that the recess 22' and cap portion 16 be of the same size, although they may be. In the illustrated embodiment, the shape 24' of the recess 22' of the second backer plate adapter 10' is a first size, and the shape 18 of the cap portion 16 of the first backer plate adapter 10 is a second size. The recess 22' of the second backer plate adapter 10' and the cap portion 16 of the first backer plate adapter 10 are adapted to engage one another. As described above, the relative sizes of the recess and cap portion may be different. However, since the second backer plate adapter 10' is used as a tool to rotate the first backer plate adapter 10, it will be understood by those skilled in the art that the sizes may only be different such that they are still functional to engage one another such that the first and second backer plate adapters 10, 10' may cooperatively rotate when engaged.

More specifically regarding the relative sizes of the recess 22' and cap portion 16, the cross-section 38' of the portion of the first end 26' taken transverse to the longitudinal axis 40' of the first end 26' exhibits a first area 44' of the recess 22' of the second backer plate adapter 10'. The cross-section 30 of the cap portion 16 of the first backer plate adapter 10, taken transverse to the longitudinal axis of the extension 12, exhibits a second area 46. In one embodiment, the first area 44' is a different size than said second area 46. in particular, the first area 44' may be larger than said second area 46. In an alternate embodiment, the first area 44' is substantially equal to the second area 46. The relative sizes of the first area 44' and second area 46, like the complementary shapes 24', 18 described above, are such as to allow for engagement and/or cooperative rotation of the first and second backer plate adapters 10, 10'.

Like the first end 26' of the second backer plate adapter 10', the first end 26 of the first backer plate adapter 10 may define a recess 22 having a shape 24 complementary to the first shape 18 of the cap portion 16 of the first backer plate adapter 10. In the illustrated embodiment, the recess 22 of the first backer plate adapter 10 has the first shape 18. Thus, the first end 26 may include at least one wall 32 defining the recess 22. More specifically, the wall 32 extends at the first end 26 of the backer plate adapter and includes an inner wall surface 34 and an outer wall surface 36. The inner wall surface 34 defines a portion of the outer boundaries of the recess 22. A cross-section 38 of the portion of the first end 26 including the wall 32 and recess 22 of the first backer plate adapter 10, taken transverse to the longitudinal axis 40 of the first backer plate adapter 10, exhibits the shape 24.

The first end 26 of the first backer plate adapter 10 is adapted to engage a syringe plunger 20. In the illustrated embodiment, the first end 26 of the first backer plate adapter 10 includes threads 50. These threads 50 are complementary to threads 52 that are disposed on the rearward face 54 of the syringe plunger 20. The first backer plate adapter 10 and syringe plunger 20 may engage one another by mating the threads 50 of the first backer plate adapter 10 with the complementary threads 52 of the syringe plunger 20. Like that of the first backer plate adapter 10, the first end 26' of the second backer plate adapter 10' may also be adapted to engage a plunger. In the illustrated embodiment, the first end 26 of the second backer plate adapter 10' includes threads 50'. These threads 50' are adapted to mate with complementary threads 52 disposed on a syringe plunger 20. As above, the configuration of the threads as shown in the illustrated embodiment is merely exemplary.

Like the first backer plate adapter 10, the second backer plate adapter 10' may include an extension 12' on its second end 28'. The extension 12' on the second end 28' of the second backer plate adapter 10' includes a shaft portion 14' and a cap portion 16'. The cap portion 16' may also have a shape 18' that is complementary to the first shape 18 of the cap portion 16 of the first backer plate adapter 10. More specifically, a cross-section 30' of the cap portion 16' taken transverse to the longitudinal axis 40 of the second backer plate adapter 10' exhibits this complementary shape 18'. In the illustrated embodiment, the cap portion 16' of the second backer plate adapter 10' has the first shape 18.

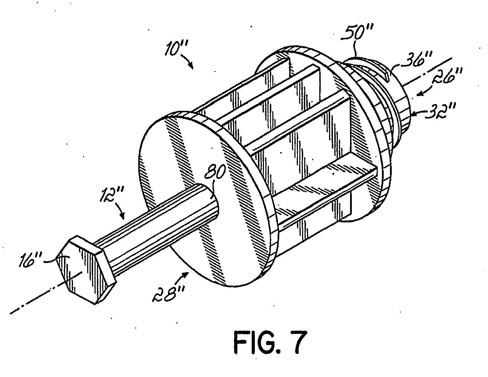

Referring now to Fig. 7, the present invention, in one embodiment, may include a backer plate adapter 10" that is adapted for use with manual injection procedures. In particular, the backer plate adapter 10" of the illustrated embodiment of Fig. 7 includes a first end 26" and a second end 28". The first end 26" of the backer plate adapter 10" is adapted to be engaged with the syringe plunger 20" (see Fig. 4). A portion of that first end 26" defines a recess 22". The second end 28" of the backer plate adapter 10" includes an extension 12" protruding therefrom. In this embodiment of the invention, the extension 12" includes an extended push rod portion 80 and a cap portion 16". Once this backer plate adapter 10" is engaged with a syringe plunger 20", at least the cap portion 16", and generally a part of the push rod portion 80, will extend from the back end of a syringe barrel to be depressed by the operator's hand in order to inject fluid from the syringe.

By use of these pluralities of backer plate adapters, the present invention also includes a method of operatively connecting a backer plate adapter to, or removing a backer plate adapter from, a syringe plunger 20. This method of operatively connecting a backer plate adapter to a syringe plunger 20 includes the following steps. First, referring to Figs. 4-6, a first backer plate adapter 10 is placed in proximity to a syringe plunger 20 such that threads 50 on the first backer plate adapter 10 are aligned to mate with threads 52 on the syringe plunger 20. In the illustrated embodiment, the first backer plate adapter 10 has a first end 26 and a second end 28. At least a portion of the first end 26 of the backer plate adapter 10 defines a recess 22. The second end 28 includes an extension 12 protruding therefrom. The extension 12 includes a shaft portion 14 and a cap portion 16, wherein a cross-section 30 of the cap portion 16 has a first shape 18. The recess 22 defined by the first end 26 has a shape 24 complementary to the first shape 18. The shape 24 of the recess 22 may be the first shape 18.

Next, one would take a second backer plate adapter 10', as described above, and use the recess 22' on the first end 26' of the second backer plate adapter 10' as a tool to engage the cap portion 16 on the extension 12 of the second end 28 of the first backer plate adapter 10. This second backer plate adapter 10', as described above and as shown in the illustrated embodiment, includes a first end 26' adapted to be engaged with a plunger, wherein a portion of the first end 26' defines a recess 22'. The recess 22' defined by the first end 26' also has a shape 24' that is complementary to the first shape 18 of the cap portion 16 of the first backer plate adapter 10. The second backer plate adapter 10' further includes a second end 28' having an extension 12' protruding therefrom. The extension 12' includes a shaft portion 14' and a cap portion 16', wherein a cross-section 30' of the cap portion 16', taken transverse to the longitudinal axis of the extension 12', may also have a complementary shape 18'.

Thus, for example, if the shape 24' of the recess 22' and the first shape 18 of the cap portion 16 are each a hexagon, the recess 22' of the second backer plate adapter 10' would be used as a hex tool to engage the hexagonal cap portion 16 of the first backer plate adapter 10. Once the recess 22' of the second backer plate adapter 10' has engaged the cap portion 16 of the extension 12 of the first backer plate adapter 10, the second backer plate adapter 10' may be rotated relative to the syringe plunger 20, thereby cooperatively rotating the first backer plate adapter 10 such that its threads 50 will mate with the complementary threads 52 of the syringe plunger 20.

In this method, and as shown in the illustrated embodiment, the first end 26 of the first backer plate adapter 10 includes first threads 50 and a portion of the syringe plunger 20 includes second threads 52. The first threads 50 are compatible with the second threads 52, and the step of positioning the first backer plate adapter 10 in engaging relationship with the syringe plunger 20 includes mating the first threads 50 with the second threads 52. This mating of the first threads 50 with the second threads 52 includes placing the first threads 50 and second threads 52 in receiving relationship with one another and rotating the first backer plate adapter 10 in a first direction relative to the syringe plunger 20.

The method of the present invention may further comprise placing the recess 22' of the second backer plate adapter 10' in receiving relationship with the cap portion 16 of the first backer plate adapter 10, and rotating the second backer plate adapter 10' in the first direction to thereby cooperatively rotate the first backer plate adapter 10 in the first direction relative to the syringe plunger 20.

When removing a backing plate from a syringe plunger 20, the method includes the use of a first backer plate adapter 10, wherein the first end 26 of the first backer plate adapter 10 Includes first threads 50 and a portion of the syringe plunger 20 includes second threads 52. The first threads 50 are compatible with the second threads 52. Moving the first backer plate adapter 10 out of engaging relationship with the syringe plunger 20 further comprises unthreading the first threads 50 from the second threads 52. Unthreading the first threads 50 from the second threads 52 may further include rotating the first backer plate adapter 10 in a direction relative to the syringe plunger.20 opposite to the first direction described above.

As described above, the first backer plate adapter 10 has a first end 26 and a second end 28. At least a portion of the first end 26 of the backer plate adapter 10 defines a recess 22. The second end 28 Includes an extension 12 protruding therefrom. The extension 12 includes a shaft portion 14 and a cap portion 16, wherein a cross-section 30 of the cap portion 16 has a first shape 18. The recess 22 defined by the first end 26 has a shape 24 complementary to the first shape 18. The shape 24 of the recess 22 may be the first shape 18.

The method of the present invention may further include a second backer plate adapter 10' including a first end 26' adapted to be engaged with a syringe plunger 20, a portion of the first end 26' defining a recess 22', and a second end 28' including an extension 12' protruding therefrom. The extension 12' includes a shaft portion 14' and a cap portion 16', wherein a cross-section 30' of the cap portion 16', taken transverse to the longitudinal axis of the extension 12' may also have a complementary shape 18'. The recess 22' defined by the first end 26' also has a shape 24' complementary to the first shape 18 of the cap portion 16 of the first backer plate adapter 10.

The method may further comprise placing the recess 22' of the second backer plate adapter 10' in receiving relationship with the cap portion 16 of the first backer plate adapter 10, and rotating the second backer plate adapter 10' in the direction opposite to the first direction to thereby cooperatively rotate the first backer plate adapter 10 in a direction opposite to the first direction relative to the syringe plunger 20.

Additional advantages and modifications will readily appear to those skilled in the art. The Invention in its broader aspects is therefore not limited to the specific details, representative apparatus and methods, and illustrative examples shown and described. Accordingly, departures may be made from such details without departing from the scope of the claims appended hereto.

## Claims

1. A backer plate adapter (10) for a syringe plunger comprising:
a first end (26) adapted to engage a syringe plunger; and
a second end (28) including an extension (12) protruding therefrom;
wherein a cross-section of at least one portion of said extension (12) taken transverse to a longitudinal axis of said extension, has a non-circular shape, **characterized in that** a portion of said first end (26) defines a recess (22), and said extension (12) of said second end (28) includes a shaft portion (14) and a cap portion (16);
wherein said cross-section is of said cap portion (16), and wherein said recess (22) has a shape that is complementary to said cap portion (16).

2. The adapter of claim 1, wherein a cross-section of said portion of said first end (26) taken transverse to a longitudinal axis of said first end exhibits a first area of said recess (22), and wherein said cross-section of said cap portion (16) exhibits a second area.

3. The adapter of claim 2, wherein said first area is greater than said second area.

4. The adapter of any of claims 1-3 wherein said first end (26) comprises outer threads (50) adapted to engage internal threads of a cavity of said plunger (20).

5. The adapter of any of claims 1-4 wherein the diameter of a body portion of said adapter is greater than the transverse dimension of said cap portion (16).

6. A plurality of adapters (10) according to any of claims 1-5, each adapter (10) comprising a cap portion (16) and a recess (22), wherein each recess (22) has a shape that is complementary to each cap portion (16).

7. An adapter according to any of claims 1-5, and further comprising:
a syringe (58) including a barrel (60); and
a syringe plunger (20) adapted to be disposed within said barrel (60), said first end (26) being adapted to engage said syringe plunger (20).

8. An adapter according to claim 7 and further comprising a second said adapter (10) for use as a tool to assemble or disassemble another said adapter (10) to or from said plunger (20).

9. A method of operating a combination of a backer plate adapter (10) and a syringe plunger (20), comprising:
providing a first said adapter (10) comprising a first end (26) and a second end (28), said first end being adapted to engage a syringe plunger, a portion of said first end defining a recess (22), and said second end including an extension (12) protruding therefrom, said extension including a shaft portion (14) and a cap portion (16), wherein a cross-section of said cap portion (16) taken transverse to a longitudinal axis of said extension has a non-circular shape, and said recess has a shape that is complementary to said non-circular shape;
providing a syringe plunger (20); and
positioning said adapter (10) in engaging relationship with said syringe plunger (20).

10. The method of claim 9, wherein said first end (26) includes male threads (50) and a portion of said syringe plunger includes female threads, said male and female threads being compatible, and wherein positioning said adapter (10) in engaging relationship with said syringe plunger (20) further comprises mating said male threads with said female threads by relative rotation.

11. The method of claim 9 comprising providing a second said adapter (10), and using said second adapter (10) as a tool to assemble or disassemble another said adapter (10) to or from said syringe plunger (20).

## Patentansprüche

1. Trägerplattenadapter (10) für einen Spritzenkolben mit
einem ersten Ende (26), das mit einem Spritzenkolben in Eingriff kommen kann;
und einem zweiten Ende (28) mit einer von diesem vorstehenden Verlängerung (12);
wobei ein Querschnitt von mindestens einem Abschnitt dieser Verlängerung (12) quer zu einer Längsachse der Verlängerung gesehen eine nicht kreisförmige Form aufweist,
**dadurch gekennzeichnet, dass** ein Abschnitt des ersten Endes (26) eine Ausnehmung (22) definiert und die Verlängerung (12) des zweiten Endes (28) einen Schaftabschnitt (14) und einen Deckelabschnitt (16) umfasst;
wobei dieser Querschnitt dem Deckelabschnitt (16) entspricht, und wobei die Ausnehmung (22) eine zu dem Deckelabschnitt (16) komplementäre Form aufweist.

2. Adapter gemäß Anspruch 1, wobei ein Querschnitt des Abschnittes des ersten Endes (26) quer zu einer Längsachse des ersten Endes gesehen einen ersten Bereich der Ausnehmung (22) aufweist, und wobei dieser Querschnitt des Deckelabschnittes (16) einen zweiten Bereich aufweist.

3. Adapter gemäß Anspruch 2, wobei der erste Bereich größer als der zweite Bereich ist.

4. Adapter gemäß einem der Ansprüche 1 bis 3, wobei das erste Ende (26) Außengewinde (50) umfasst, die mit Innengewinden eines Hohlraumes des Kolbens (20) in Eingriff kommen können.

5. Adapter gemäß einem der Ansprüche 1 bis 4, wobei der Durchmesser eines Körperabschnittes des Adapters größer ist als die Querabmessung des Deckelabschnittes (16).

6. Mehrere Adapter (10) gemäß einem der Ansprüche 1 bis 5, wobei jeder Adapter (10) einen Deckelabschnitt (16) und eine Ausnehmung (22) umfasst, und wobei jede Ausnehmung (22) eine zu jedem Deckelabschnitt (16) komplementäre Form aufweist.

7. Adapter gemäß einem der Ansprüche 1 bis 5, der weiterhin Folgendes umfasst:
eine Spritze (58) mit einem Zylinder (60); und
einen Spritzenkolben (20), der in dem Zylinder (60) angeordnet werden kann,
wobei das erste Ende (26) mit dem Spritzenkolben (20) in Eingriff gebracht werden kann.

8. Adapter gemäß Anspruch 7, der weiterhin einen zweiten Adapter (10) zur Verwendung als Werkzeug umfasst, um einen weiteren Adapter (10) an dem Kolben (20) anzubringen oder von diesem abzunehmen.

9. Verfahren zum Betreiben einer Kombination aus einem Trägerplattenadapter (10) und einem Spritzenkolben (20) mit den Schritten:
Vorsehen eines ersten Adapters (10) mit einem ersten Ende (26) und einem zweiten Ende (28), wobei das erste Ende mit einem Spritzenkolben in Eingriff kommen kann, wobei ein Abschnitt des ersten Endes eine Ausnehmung (22) definiert und das zweite Ende eine von diesem vorstehende Verlängerung (12) aufweist, wobei die Verlängerung einen Schaftabschnitt (14) und einen Deckelabschnitt (16) umfasst, wobei ein Querschnitt des Deckelabschnittes (16) quer zu einer Längsachse der Verlängerung gesehen eine nicht kreisförmige Form hat und die Ausnehmung eine zu der nicht kreisförmigen Form komplementäre Form aufweist;
Vorsehen eines Spritzenkolbens (20); und
Anordnen des Adapters (10) in Eingriffbeziehung mit dem Spritzenkolben (20).

10. Verfahren gemäß Anspruch 9, wobei das erste Ende (26) Außengewinde (50) umfasst und ein Abschnitt des Spritzenkolbens Innengewinde umfasst, wobei die Außen- und Innengewinde kompatibel sind, und wobei die Anordnung des Adapters (10) in Eingriffsbeziehung mit dem Spritzenkolben (20) weiterhin eine Verbindung dieser Außengewinde mit den Innengewinden durch eine relative Drehung umfasst.

11. Verfahren gemäß Anspruch 9, mit den Schritten: Vorsehen eines zweiten Adapters (10) und Verwenden des zweiten Adapters (10) als Werkzeug, um einen weiteren Adapter (10) an dem Spritzenkolben (20) anzubringen oder von diesem abzunehmen.

## Revendications

1. Adaptateur de plaque d'appui (10) pour un piston de seringue comprenant :
une première extrémité (26) adaptée pour venir en prise avec un piston de seringue; et
une seconde extrémité (28) comprenant une extension (12) faisant saillie à partir de celle-ci :
dans lequel une coupe transversale d'au moins une partie de ladite extension (12) prise de façon transversale par rapport à un axe longitudinal de ladite extension, possède une forme non circulaire, **caractérisée en ce qu'**une partie de ladite première extrémité (26) définit un évidement (22), et ladite extension (12) de ladite seconde extrémité (28) comprend une partie de tige (14) et une partie de capuchon (16) ;
dans lequel ladite coupe transversale se compose de ladite partie de capuchon (16), et dans lequel ledit évidement (22) présente une forme qui est complémentaire à ladite partie de capuchon (16).

2. Adaptateur selon la revendication 1, dans lequel une coupe transversale de ladite partie de ladite première extrémité (26) prise de façon transversale par rapport à un axe longitudinale de ladite première extrémité présente une première section dudit évidement (22), et dans lequel ladite coupe transversale de ladite partie de capuchon (16) présente une seconde section.

3. Adaptateur selon la revendication 2, dans lequel ladite première section est supérieure à ladite seconde section.

4. Adaptateur selon l'une quelconque des revendications 1 à 3, dans lequel ladite première extrémité (26) comprend un filetage externe (50) adapté pour venir en prise avec un filetage interne d'une cavité dudit piston (20).

5. Adaptateur selon l'une quelconque des revendications 1 à 4, dans lequel le diamètre d'une partie du corps dudit adaptateur est supérieur à la dimension transversale de ladite partie de capuchon (16).

6. Une pluralité d'adaptateurs (10) selon l'une quelconque des revendications 1 à 5, chaque adaptateur (10) comprenant une partie de capuchon (16) et un évidement (22), dans lequel chaque évidement (22) présente une forme qui est complémentaire à chaque partie de capuchon (16).

7. Adaptateur selon l'une quelconque des revendications 1 à 5 et comprenant en outre :
une seringue (58) comprenant un cylindre (60) ; et
un piston de seringue (20) adapté pour être disposé dans ledit cylindre (60), ladite première extrémité (26) étant adaptée pour venir en prise avec ledit piston de seringue (20).

8. Adaptateur selon la revendication 7, et comprenant en outre un second dit adaptateur (10) destiné à être utilisé comme un outil pour assembler ou désassembler un autre dit adaptateur (10) sur ou à partir dudit piston (20).

9. Procédé consistant à faire fonctionner une combinaison d'adaptateur de plaque d'appui (10) et de piston de seringue (20), comprenant :
la fourniture du premier dit adaptateur(10) comprenant une première extrémité (26) et une seconde extrémité (28), ladite première extrémité étant adaptée pour venir en prise avec un piston de seringue, une partie de ladite première extrémité définissant un évidement (22), et ladite seconde extrémité comprenant une extension (12) faisant saillie à partir de celle-ci, ladite extension comprenant une partie de tige (14) et une partie de capuchon (16), dans lequel une coupe transversale de ladite partie de capuchon (16) prise de façon transversale par rapport à un axe longitudinal de ladite extension présente une forme non circulaire, et ledit évidement possède une forme qui est complémentaire à ladite forme non circulaire;
la fourniture d'un piston de seringue (20) ; et
la fourniture dudit adaptateur (10) dans un rapport d'enclenchement avec ledit piston de seringue (20).

10. Procédé selon la revendication 9, dans lequel ladite première extrémité (26) comprend un filetage mâle (50) et une partie dudit piston de seringue comprend un filetage femelle, lesdits filetages mâle et femelle étant compatibles, dans lequel le positionnement dudit adaptateur (10) dans un rapport d'enclenchement avec ledit piston de seringue (20) comprend en outre l'accouplement dudit filetage mâle avec ledit filetage femelle par rotation relative.

11. Procédé selon la revendication 9 comprenant la fourniture d'un second dit adaptateur (10) et l'utilisation dudit second adaptateur (10) comme un outil pour assembler ou désassembler un autre dit adaptateur (10) sur ou à partir dudit piston de seringue (20).
